# FASCICULE DE BREVET EUROPEEN

(11) **EP 1 587 488 B1**
(45) Date de publication et mention de la délivrance du brevet: **13.09.2006**
(21) Numéro de dépôt: 04701986.4
(22) Date de dépôt: 14.01.2004
(51) Int. Cl.: A61K 8/64, A61K 8/81, A61K 8/44, A61K 8/33, A61Q 5/10

(54) **COMPOSITION POUR LA COLORATION PERMANENTE DE FIBRES KERATINIQUES**
ZUSAMMENSETZUNG ZUM PERMANENTEN FÄRBEN VON KERATINFASERN
COMPOSITION FOR PERMANENT DYEING OF KERATINOUS FIBERS

(30) Priorité: 15.01.2003 FR 0300409
(43) Date de publication de la demande: 26.10.2005
(73) Titulaire: Eugene Perma France, 75009 Paris (FR)
(72) Inventeur: DUCHERON, Gérard, F-94360 BRIE SUR MARNE (FR); JAUFFRET, Hélène, F-92600 ASNIERES SUR SEINE (FR)
(74) Mandataire: Catherine, Alain
(86) Numéro de dépôt international: PCT/FR2004/050015
(87) Numéro de publication internationale: WO 2004/064794

(56) Documents cités:
- EP-A- 0 821 935
- WO-A-01/47480
- WO-A-01/97757

## Description

La présente invention a pour objet une composition cosmétique pour la coloration permanente de fibres kératiniques, et en particulier des fibres capillaires.

Cette composition a pour propriété d'atténuer les effets délétères du procédé chimique mis en jeu lors de la coloration permanente des fibres capillaires, et de préserver, lors de la coloration, les propriétés naturelles du cheveu.

Les seuls procédés de coloration capillaire capables de couvrir parfaitement et durablement les cheveux sont les procédés de coloration par oxydation qui aboutissent à des colorations dites permanentes.

Ces procédés mettent en jeu l'action combinée d'un agent oxydant, en général le peroxyde d'hydrogène, et d'une base alcaline, préférentiellement l'ammoniaque. Cette combinaison a pour effet d'induire simultanément, la décoloration de la pigmentation naturelle ou artificielle des cheveux, et l'oxydation des précurseurs de colorants, avec pour résultat la création d'une nouvelle nuance colorée.

La base alcaline, préférentiellement l'ammoniaque, facilite la dissolution des colorants et, par alcalinisation du milieu, favorise l'action décolorante du peroxyde d'hydrogène en participant à la libération de l'oxygène actif.

L'ammoniaque agit aussi sur le gonflement de la fibre en provoquant l'ouverture des écailles. Elle facilite ainsi la pénétration des précurseurs de colorants et assure une répartition homogène des pigments jusqu'au coeur de la fibre.

L'agent oxydant, en général le peroxyde d'hydrogène, a deux fonctions : décolorer les pigments en place afin d'éviter les variations de teinture résultant de la couleur initiale du cheveu, et déclencher le processus oxydatif des précurseurs de colorants. Dans une première étape, ces précurseurs se transforment en intermédiaires très réactifs qui se couplent entre eux pour former, dans une deuxième étape de condensation oxydative, des copolymères colorés pouvant se fixer solidement dans la fibre kératinique.

Cette technique de coloration permanente donne d'excellents résultats, tant en ce qui concerne la couverture des cheveux blancs (proche de 100%), la variété des nuances offertes et la tenue relative de la couleur dans le temps.

Elle présente néanmoins plusieurs inconvénients, dont celui de provoquer rapidement des dégradations physico-chimiques importantes sur la fibre.

Du fait de la croissance régulière des fibres dans leur teinte naturelle d'origine qui provoque une démarcation inesthétique avec les longueurs colorées artificiellement, et de l'élimination progressive de la coloration, des applications régulières de teinture sont nécessaires.

Soumise aux conditions alcaline-oxydantes extrêmes mises en jeu lors du processus, la fibre, chimiquement et physiquement altérée dans son intégrité, perd progressivement ses propriétés naturelles. Inéluctablement au fil des colorations, les cheveux deviennent secs et rêches au toucher et perdent leur souplesse et leur brillance naturelles. La résistance de la couleur à l'élimination diminue. De ce fait, les produits de coloration semblent manquer de performance. Le document WO-A-0197757 décrit des compositions pour la coloration permanente des cheveux comprenant ou précurseur, un agent alcalin, un oxydant, des hydrolysats de protéines et des copolymères d'acide acrylique et diméthylammonium.

La demanderesse a découvert que l'addition d'un complexe quaternaire spécifique aux bases colorantes communément utilisées permettait de colorer durablement et efficacement les cheveux (tenue de la couleur renforcée), tout en préservant leurs propriétés mécaniques naturelles (résistance et souplesse) et en améliorant leurs propriétés cosmétiques (douceur et brillance).

La présente invention a donc pour objet une composition pour la coloration permanente des fibres kératiniques, et en particulier des cheveux, comprenant au moins un composé oxydant, au moins un précurseur de colorants et au moins un agent alcalin, caractérisée en ce qu'elle comprend en outre :
- un hydrolysat de protéine,
- un copolymère quaternisé de di-méthylallyl ammonium et d'acide acrylique,
- un di-alkyl ester de N méthyl triéthanolamine méthylsulfate, et
- un di-alkyl carbonate.

De manière avantageuse, l'hydrolysat de protéine, le copolymère quaternisé, le di-alkyl ester de N méthyl triéthanolamine méthylsulfate et le di-alkyl carbonate forment un complexe associatif quaternaire au sein de la composition.

De préférence, l'hydrolysat de protéine est un hydrolysat de séricine, une protéine de la soie.

L'hydrolysat de séricine est un mélange, concentré et purifié, de peptides de taille moléculaire inférieure ou égale à 20 kDa. Il est caractérisé par une forte teneur en sérine et en acide aspartique. Idéalement, il peut être constitué de 18 acides aminés, dont la plupart présentent des groupements latéraux fortement polaires tels que des groupes hydroxyle, carboxyle et aminés.

L'hydrolysat de protéine représente de préférence de 0,1 à 10%, mieux de 1 à 5%, en poids du poids total de la composition.

Le copolymère quaternisé de di-méthylallyl ammonium et d'acide acrylique compris dans la composition selon l'invention correspond en général à la formule I suivante : 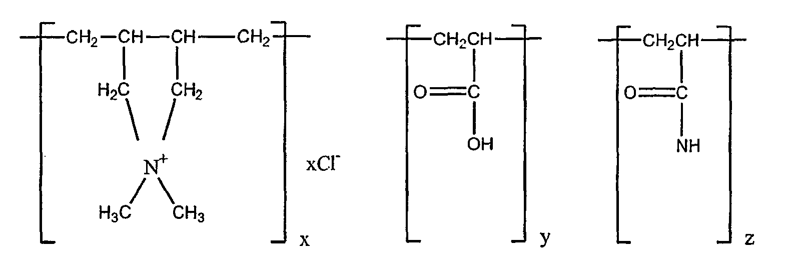 dans laquelle x est compris entre 1 et 1000, de préférence entre 1 et 100, et y et z sont compris indépendamment entre 0 et 1000, de préférence entre 0 et 100.

Avantageusement, le copolymère quaternisé est un polyquaternium, par exemple un polyquaternium 22 (nom CTFA).

Le copolymère quaternisé de di-méthylallyl ammonium et d'acide acrylique représente de préférence de 0,1 à 1,5%, mieux de 0,5 à 1%, en poids du poids total de la composition.

Le di-alkyl ester de N méthyl triéthanolamine méthylsulfate compris dans la composition selon l'invention correspond en général à la formule II suivante : 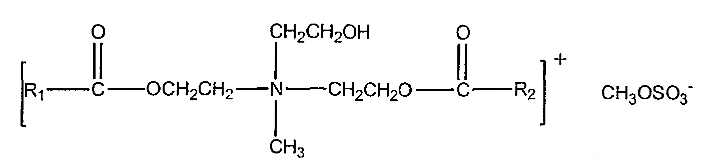 dans laquelle R₁ et R₂ sont des groupes alkyles ou alcényles en Cn, où n est compris entre 1 et 100, de préférence entre 6 et 20.

Les groupes alkyles utilisables pour R₁ et R₂ peuvent être des chaînes caproyle, caprylyle, capryle, lauryle, myristyle, cétyle, stéaryle, arachidyle ou béhényle. Les groupes alcényles utilisables pour R₁ et R₂ peuvent être des chaînes caproléyle, lauroléyle, myristoléyle, palmitoléyle, oléyle, linoléyle, linolényle, arachidonyle ou érucyle.

Avantageusement, les groupes R₁ et R₂ représentent un groupe alkyle dérivé de l'huile de noix de coco, en particulier une chaîne lauryle. Ainsi, le di-alkyl ester utilisé de préférence est un dicocoyl ester de N méthyl triéthanolamine méthylsulfate.

Le di-alkyl ester de N méthyl triéthanolamine méthylsulfate représente de préférence de 0,1 à 5%, mieux de 0,5 à 2%, en poids du poids total de la composition.

Le di-alkyl carbonate compris dans la composition selon l'invention correspond généralement à la formule III suivante : 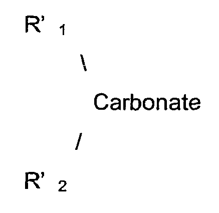 dans laquelle R₁' et R₂' sont des groupes alkyles ou alcényles en Cn, linéaires ou ramifiés, où n est compris entre 1 et 100, de préférence entre 6 et 20.

Les groupes alkyles utilisables pour R'₁ et R'₂ peuvent être des chaînes caproyle, caprilyle, capryle, lauryle, myristyle, cétyle, stéaryle, arachidyle ou béhényle. Les groupes alcényles utilisables pour R'₁ et R'₂ peuvent être des chaînes caproléyle, lauroléyle, myristoléyle, palmitoléyle, oléyle, linoléyle, linolényle, arachidonyle ou érucyle.

Avantageusement, les groupes R₁' et R₂' représentent une chaîne caprilyle, le di-alkyl carbonate utilisé étant ainsi le di-caprilyl carbonate.

Le di-alkyl carbonate représente de préférence de 0,02 à 2,5%, mieux de 0,1 à 1 %, en poids du poids total de la composition.

Ainsi, la composition selon l'invention peut contenir de 0,32 à 19% en poids, et préférentiellement de 2,1 à 9% en poids du complexe associatif quaternaire formé par les quatre composés identifiés ci-dessus.

Outre les quatre composés décrits précédemment, la composition selon l'invention comprend au moins un agent alcalin, au moins un précurseur de colorants et au moins un composé oxydant.

Le ou les agents alcalins présents dans la composition selon l'invention peuvent être choisis parmi l'aminométhylpropanol, l'ammoniaque, la monoéthanolamine, la diéthanolamine, la triéthanolamine et leurs mélanges.

Le ou les agents alcalins représentent de préférence de 1 à 30%, mieux de 1 à 4%, en poids du poids total de la composition. En particulier, la composition selon l'invention comprend avantageusement entre 1 et 4% en poids d'ammoniaque.

Le ou les précurseurs de colorants d'oxydation présents dans la composition selon l'invention sont généralement des composés aromatiques tels que les diamines aromatiques, les aminophénols ou les phénols. Ils peuvent être choisis parmi les bases et les coupleurs.

Les bases ou intermédiaires primaires peuvent être des amines aromatiques, des diaminophénols ou des aminophénols dont les groupes NH₂ et OH sont en position ortho ou para les uns par rapport aux autres. On peut citer notamment la para-phénylène diamine (pPD), l'ortho-aminophénol (oAP), le para-méthylamino phénol (pMAP), le para-amino phénol (pAP), la para-toluylène diamine (pTD) et la N phényl paraphénylène diamine (NpPD).

Les coupleurs ou modificateurs peuvent être des méta-diamines, des méta-aminophénols, des polyphénols ou des naphtols. On peut citer notamment le méta-aminophénol (mPD), le résorcinol (R), le 1-naphtol (1-N), la méta-phénylène diamine (mPD), le para-amino ortho-crésol (pAOC), le 1-5 dihydroxynaphtalène (1-5 DHN) et/ou le 2-7 dihydroxynaphtalène (2-7 DHN).

Le ou les précurseurs de colorants représentent de préférence de 0,5 à 10%, mieux de 1 à 2,5%, en poids du poids de la composition.

La composition selon l'invention comprend enfin au moins un composé oxydant. Un composé oxydant particulièrement préféré est le peroxyde d'hydrogène.

Avantageusement, le ou les composés oxydants sont introduits dans la composition selon l'invention sous la forme d'une solution, à une teneur de préférence comprise entre 1,5 et 12%, mieux entre 6 et 9%, en poids du poids de la solution. Dans ce cas, la solution représente de préférence 50% en poids du poids total de la composition. Ainsi, la teneur en composés oxydants dans la composition selon l'invention est comprise de préférence entre 0,75 et 6%, mieux entre 3 et 4,5%, en poids du poids total de la composition.

Le pH de la composition est compris de préférence entre 7 et 11, mieux entre 8,5 et 10,5.

Ainsi, de manière avantageuse, la composition selon la présente invention comprend :
- entre 0,1 et 10% en poids d'hydrolysat de protéine, de préférence d'hydrolysat de séricine,
- entre 0,1 et 1,5% en poids de copolymère quaternisé de diméthylallyl ammonium et d'acide acrylique,
- entre 0,1 et 5% en poids de di-alkyl ester de N méthyl triéthanolamine méthylsulfate,
- entre 0,02 et 2,5% en poids de di-alkyl carbonate,
- entre 0,75 et 6% en poids d'au moins un composé oxydant,
- entre 0,5 et 10% en poids d'au moins un précurseur de colorants, et
- une quantité suffisante d'au moins un agent alcalin pour atteindre un pH compris entre 7 et 11, de préférence entre 8,5 et 10,5.

Mieux, la composition selon la présente invention comprend :
- entre 1 et 5% en poids d'hydrolysat de protéine, de préférence d'hydrolysat de séricine,
- entre 0,5 et 1 % en poids de copolymère quaternisé de diméthylallyl ammonium et d'acide acrylique,
- entre 0,5 et 2% en poids de di-alkyl ester de N méthyl triéthanolamine méthylsulfate,
- entre 0,1 et 1 % en poids de di-alkyl carbonate,
- entre 3 et 4,5% en poids d'au moins un composé oxydant,
- entre 1 et 2,5% en poids d'au moins un précurseur de colorants, et
- une quantité suffisante d'au moins un agent alcalin pour atteindre un pH compris entre 7 et 11, de préférence entre 8,5 et 10,5.

La composition selon l'invention peut en outre contenir différents adjuvants usuels des colorations d'oxydation. Ces adjuvants peuvent être des acides gras, naturels ou synthétiques, des alcools gras, naturels ou synthétiques, oxyéthylénés ou polyglycérolés, des huiles minérales ou végétales, des agents anti-oxydants, des séquestrants et des parfums.

Les acides gras utilisables comme adjuvants dans la composition selon l'invention, comportent généralement de 10 à 18 atomes de carbone, et représentent de préférence de 1 à 20%, mieux de 5 à 15%, en poids du poids total de la composition.

Les alcools gras utilisables comme adjuvants dans la composition selon l'invention, peuvent être choisis parmi l'alcool oléique, l'alcool laurique, l'alcool myristique, l'alcool cétylique, l'alcool stéarylique, le ceteareth 33 et leurs mélanges. Ils représentent de préférence de 1 à 25%, mieux de 5 à 20%, en poids du poids total de la composition.

Comme huile utilisable comme adjuvant dans la composition selon l'invention, on peut citer l'huile de Coprah.

Comme agent anti-oxydant utilisable comme adjuvant dans la composition selon l'invention, on peut citer le métabisulfite de sodium et le 1-phényl-3-méthyl-5-pyrazolone.

La composition selon l'invention se présente généralement sous la forme d'une crème ou d'un gel, de préférence sous la forme d'une crème.

La composition objet de la présente invention peut être fabriquée par mélange, d'une manière connue de l'homme du métier, des différents constituants qu'elle comprend.

L'invention a encore pour objet un complexe associatif quaternaire pour la coloration de fibres kératiniques, comprenant :
- un hydrolysat de protéine, de préférence un hydrolysat de séricine,
- un copolymère quaternisé de di-méthylallyl ammonium et d'acide acrylique,
- un di-alkyl ester de N méthyl triéthanolamine méthylsulfate, et
- un di-alkyl carbonate.

L'invention a également pour objet une base, se présentant sous la forme d'un gel ou d'une crème, pour la fabrication d'une composition telle que définie précédemment, qui comprend :
- un hydrolysat de protéine, de préférence un hydrolysat de séricine,
- un copolymère quaternisé de di-méthylallyl ammonium et d'acide acrylique,
- un di-alkyl ester de N méthyl triéthanolamine méthylsulfate,
- un di-alkyl carbonate,
- au moins un précurseur de colorants, et
- au moins un agent alcalin.

L'invention a également pour objet une solution pour la fabrication d'une composition telle que définie précédemment, qui comprend au moins un composé oxydant, de préférence le peroxyde d'hydrogène.

Avantageusement, le ou les composés oxydants sont présents dans la solution, à une teneur de préférence comprise entre 1,5 et 12%, mieux entre 6 et 9%, en poids du poids de la solution.

L'invention a également pour objet un procédé pour révéler la coloration d'une base telle que définie précédemment, qui comprend l'étape de mélange de la base définie précédemment avec la solution définie précédemment, de préférence dans un rapport 1/1 en poids.

L'invention a enfin pour objet un procédé de coloration de fibres kératiniques, de préférence de cheveux, qui comprend les étapes suivantes :
- application sur lesdites fibres de la composition selon l'invention, pendant une durée permettant d'obtenir la coloration recherchée, en général pendant 10 à 45 minutes, de préférence de l'ordre de 30 minutes.
- rinçage et séchage des fibres.

La composition selon l'invention permet de colorer les cheveux avec une couverture et une solidité supérieure à celles des compositions communément utilisées. Les colorations couvrent parfaitement et uniformément les cheveux, sans surcharge sur les zones sensibilisées, et montrent une stabilité aux shampooings nettement améliorée. Les nuances conservent leur éclat et les reflets, plus intenses le jour de la coloration, ne perdent pas leur luminosité au fur et à mesure des shampooings.

Une hypothèse pouvant être formulée, sans pour autant que le déposant y soit lié, est que le complexe formé améliore la pénétration et la fixation des colorants dans la fibre. De plus, du fait de leur caractère cationique, les composés rentrant dans la composition selon l'invention présentent une forte affinité pour la kératine et forment à la surface de la fibre un film qui aurait un rôle protecteur et réparateur pendant la réaction oxydative.

Protégés par la composition selon l'invention, les cheveux gardent après la coloration un toucher doux et soyeux. Ils conservent la souplesse, la résistance, l'élasticité et la brillance d'un cheveu sain et naturel.

Les propriétés hydratantes et apaisantes des fractions peptidiques du complexe présentent également l'avantage de limiter de façon significative la diversité et l'intensité des réactions d'inconfort cutané qui accompagnent généralement l'application des produits de coloration d'oxydation.

L'invention sera mieux comprise par les exemples suivants non limitatifs.

### EXEMPLE 1: Préparation d'une crème de coloration permanente de nuance châtain.

On formule une composition de coloration selon l'invention à partir d'une base et d'une solution comprenant un composé oxydant.

La formule de la base est la suivante, en grammes :
- Alcool cétyl stéarylique 9
- Alcool cétéarylique/Ceteareth 33 11,5
- Acide oléique 5
- Huile de Coprah 1,5
- Métabisulfite de sodium 0,6
- Sel sodique de l'acide pentétique 1
- Hydrolysat de séricine 3 (SETAKOL, commercialisé par la société PENTAPHARM)
- Di n-octyl carbonate 1
- Di-cocoyléthyl Hydroxyéthylmonium Méthylsulfate (DEHYQUART L80, commercialisé par la société COGNIS) 1,5
- Copolymère acrylique/chlorure de diméthyl diallyl ammonium (MERQUAT 280 (polyquaternium 22) commercialisé par la société ONDEO NALCO) 1,5
- Ammoniaque 12
- Méta-phénylène diamine 0,04
- Résorcinol 0,6
- Para-phénylène diamine 0,5
- Para-amino phéno l 0,07
- Méta-amino phénol 0,09
- 1-phényl-3-méthyl-5-pyrazolone 0,1
- Parfum 0,4
- Eau déminéralisée qsp 100

Au moment de l'emploi, cette base est diluée avec 100g d'eau oxygénée à 20 volumes (c'est-à-dire à 6% en poids de peroxyde d'hydrogène), pour donner une composition colorante selon l'invention, qui est alors appliquée pendant 30 minutes sur les cheveux.

### EXEMPLE 2 : Préparation d'une crème de coloration permanente de nuance Blond.

On formule une composition de coloration selon l'invention à partir d'une base et d'une solution comprenant un composé oxydant. La formule de la base est la suivante, en grammes :
- Alcool cétyl stéarylique 9
- Alcool cétéarylique /Ceteareth 33 11,5
- Acide oléique 5
- Huile de Coprah 1,5
- Métabisulfite de sodium 0,6
- Sel sodique de l'acide pentétique 1
- Hydrolysat de séricine (SETAKOL, commercialisé par la société PENTAPHARM) 3
- Di n-octyl carbonate 1
- Di-cocoyléthyl Hydroxyéthylmonium Méthylsulfate (DEHYQUART L80 commercialisé par la société COGNIS) 1,5
- Copolymère acrylique/chlorure de diméthyl diallyl ammonium (MERQUAT 280 (polyquaternium 22) commercialisé par la société ONDEO NALCO) 1,5
- Ammoniaque 12
- Résorcinol 0,2
- Para-phénylène diamine 0,15
- Para-amino phénol 0,18
- Méta-amino phénol 0,08
- 1-phényl-3-méthyl-5-pyrazolone 0,1
- Ortho-aminophénol 0,03
- NNB HE para phénylène diamine sulfate 0,1
- Parfum 0,4
- Eau déminéralisée qsp 100

Au moment de l'emploi, cette base est diluée avec 100g d'eau oxygénée à 20 volumes, pour donner une composition colorante selon l'invention, qui est alors appliquée pendant 30 minutes sur les cheveux.

### EXEMPLE 3 : Résistance aux shampooings de la coloration selon l'invention

La résistance de la coloration aux shampooings est quantifiée par la mesure de la variation de la couleur générée par six shampooings successifs sur des coupons de laine ayant subi une coloration (nuance châtain clair) à l'aide :
- d'une composition selon l'invention renfermant un complexe associatif quaternaire, ou
- d'une composition ne renfermant pas de complexe associatif quaternaire (c'est à dire ne renfermant aucun des 4 constituants du complexe associatif quaternaire), ou
- d'une composition renfermant une association binaire d'actifs A, ou
- d'une composition renfermant une association binaire d'actifs B.

Chaque composition est formulée à partir d'une base colorante et d'une solution comprenant un composé oxydant.

Les formulations des 4 bases colorantes utilisées sont les suivantes :

### Base colorante selon l'invention avec complexe associatif quaternaire

- Alcool cétyl stéarylique 9
- Alcool cétéarylique/Ceteareth 33 11,5
- Acide oléique 5
- Huile de Coprah 1,5
- Métabisulfite de sodium 0,6
- Sel sodique de l'acide pentétique 1
- Hydrolysat de séricine (SETAKOL commercialisé par la société PENTAPHARM) 3
- Di n-octyl carbonate 1
- Di-cocoyléthyl Hydroxyéthylmonium Méthylsulfate (DEHYQUART L80 commercialisé par la société COGNIS) 1,5
- Copolymère acrylique/chlorure de diméthyl diallyl ammonium (MERQUAT 280 (polyquaternium 22) commercialisé par la société ONDEO NALCO) 1,5
- Ammoniaque 12
- Méta-phénylène diamine 0,035
- Résorcinol 0,5
- Para-phénylène diamine 0,4
- Para-amino phénol 0,05
- Méta-amino phénol 0,07
- 1-phényl-3-méthyl-5-pyrazolone 0,1
- Parfum 0,4
- Eau déminéralisée qsp 100

### Base colorante sans complexe associatif quaternaire

- Alcool cétyl stéarylique 9
- Alcool cétéarylique/Ceteareth 33 11,5
- Acide oléique 5
- Huile de Coprah 1,5
- Métabisulfite de sodium ,6
- Sel sodique de l'acide pentétique 1
- Ammoniaque 12
- Méta-phénylène diamine 0,035
- Résorcinol 0,5
- Para-phénylène diamine 0,4
- Para-amino phénol 0,05
- Méta-amino phénol 0,07
- 1-phényl-3-méthyl-5-pyrazolone 0,1
- Parfum 0,4
- Eau déminéralisée qsp 100

### Base colorante avec association binaire A

- Alcool cétyl stéarylique 9
- Alcool cétéarylique/Ceteareth 33 11,5
- Acide oléique 5
- Huile de Coprah 1,5
- Métabisulfite de sodium 0,6
- Sel sodique de l'acide pentétique 1
- Hydrolysat de séricine (SETAKOL commercialisé par la société PENTAPHARM) 3
- Copolymère acrylique/chlorure de diméthyl diallyl ammonium (MERQUAT 280 (polyquaternium 22) commercialisé par la société ONDEO NALCO) 1,5
- Ammoniaque 12
- Méta-phénylène diamine 0,035
- Résorcinol 0,5
- Para-phénylène diamine 0,4
- Para-amino phénol 0,05
- Méta-amino phénol 0,07
- 1-phényl-3-méthyl-5-pyrazolone 0,1
- Parfum 0,4
- Eau déminéralisée qsp 100

### Base colorante avec association binaire B

- Alcool cétyl stéarylique 9
- Alcool cétéarlylique/Ceteareth 33 11,5
- Acide oléique 5
- Huile de Coprah 1,5
- Métabisulfite de sodium 0,6
- Sel sodique de l'acide pentétique 1
- Di n-octyl carbonate 1
- Di-cocoyléthyl Hydroxyéthylmonium Méthylsulfate (DEHYQUART L80 commercialisé par la société COGNIS) 1,5
- Ammoniaque 12
- Méta-phénylène diamine 0,035
- Résorcinol 0,5
- Para-phénylène diamine 0,4
- Para-amino phénol 0,05
- Méta-amino phénol 0,07
- 1-phényl-3-méthyl-5-pyrazolone 0,1
- Parfum 0,4
- Eau déminéralisée qsp 100

Au moment de l'emploi, chacune de ces bases colorantes est diluée avec 100g d'eau oxygénée à 20 volumes, pour donner une composition colorante, qui est alors appliquée pendant 30 minutes sur les coupons de laine.

Les mesures, en espace couleur L*a* b*, sont réalisées à l'aide d'un colorimètre MINOLTA CR 210.

La tenue de la couleur est quantifiée par la valeur de l'écart couleur ΔE*ab, définie par l'équation suivante: ΔE*ab = [(ΔL*)2 + (Δa*) + (Δb*)2 ]^{½}.

La diminution de la valeur ΔE est proportionnelle à l'amélioration de la tenue de la couleur.

Les résultats obtenus figurent dans le tableau 1.

**Tableau 1**

| COMPOSITION | ΔE après 6 shampooings |
|---|---|
| Sans complexe associatif quaternaire | 12,81 |
| Avec complexe associatif quaternaire | 5,61 |
| Avec association binaire A | 10,69 |
| Avec association binaire B | 10,4 |

Après 6 shampooings, les tissus de laine colorés par la composition ne renfermant pas le complexe associatif quaternaire présentent une variation couleur de 12,81 contre 5,61 seulement pour les tissus colorés avec la composition selon l'invention renfermant le complexe.

La présence du complexe associatif quaternaire améliore donc la résistance de la coloration au lavage.

En comparaison, l'association binaire des constituants du complexe associatif quaternaire améliore la résistance de la couleur à l'élimination aux shampooings, mais de façon nettement moins marquée que dans le cas du complexe quaternaire.

II y a donc une réelle synergie entre les quatre actifs qui composent le complexe associatif quaternaire selon l'invention.

### EXEMPLE 4 : Résistance à l'action des rayonnements ultraviolets

La résistance de la coloration aux rayonnements ultraviolets est quantifiée par la mesure de la variation de la couleur (ΔE) générée par l'exposition à une irradiation intense de coupons de laine ayant subi une coloration (nuance châtain clair) à l'aide :
- d'une composition selon l'invention renfermant un complexe associatif quaternaire, ou
- d'une composition ne renfermant pas de complexe associatif quaternaire (c'est à dire ne renfermant aucun des 4 constituants du complexe associatif quaternaire), ou
- d'une composition renfermant une association binaire d'actifs A, ou
- d'une composition renfermant une association binaire d'actifs B.

Les formulations de ces compositions sont identiques à celles des compositions de coloration utilisées dans l'exemple 3.

Les mesures, en espace couleur L*a*b* sont réalisées à l'aide d'un colorimètre MINOLTA CR 210.

L'irradiation est réalisée à l'aide d'un appareil SUNTEST, les coupons de laine étant exposés à un rayonnement lumineux intense pendant 40 heures.

Les résultats obtenus figurent dans le tableau 2.

**TABLEAU 2**

| Composition | ΔE après 6 shampooings |
|---|---|
| Sans complexe associatif quaternaire | 8,17 |
| Avec complexe associatif quaternaire | 5,52 |
| Avec association binaire A | 8,01 |
| Avec association binaire B | 7,4 |

La présence du complexe associatif quaternaire permet d'améliorer de façon importante la stabilité de la couleur sous une irradiation ultraviolette.

La synergie des quatre actifs est une nouvelle fois mise en évidence, la coloration présentant une meilleure stabilité en présence des quatre composants du complexe associatif quaternaire selon l'invention.

### EXEMPLE 5: Effet protecteur du complexe associatif quaternaire.

L'effet protecteur du complexe associatif quaternaire est quantifié par la mesure des propriétés mécaniques (module d'élasticité à 5% d'allongement) des cheveux après coloration à l'aide :
- d'une composition selon l'invention renfermant un complexe associatif quaternaire, ou
- d'une composition selon l'invention ne renfermant pas un complexe associatif quaternaire (c'est à dire ne renfermant aucun des 4 constituants du complexe associatif quaternaire).

Les formulations de ces deux compositions sont identiques aux deux compositions correspondantes de l'exemple 3.

Les mesures sont réalisées à l'aide d'un DYNAMOMETRE LLOYD LRY relié à un ordinateur.

Le module d'élasticité d'une mèche est obtenu en mesurant la pente de la courbe force/allongement (en Newton/% allongement). Le rapport des deux modules mesurés en présence et en absence du complexe permet d'évaluer l'effet dudit complexe sur les propriétés mécaniques des cheveux. Les résultats figurent dans le tableau 3.

**TABLEAU 3**

| Composition | Module d'élasticité (Newton/5% allongement) | % d'amélioration |
|---|---|---|
| Sans complexe associatif quaternaire | 3,41 | 7 |
| Avec complexe associatif quaternaire | 3,19 | |

Les valeurs de module sont significativement différentes au risque 5%. Le complexe quaternaire permet d'améliorer de 7% les propriétés élastiques des cheveux après coloration avec la composition selon l'invention, ce qui montre les propriétés protectrices du complexe associatif quaternaire selon l'invention.

## Revendications

1. Composition pour la coloration permanente de fibres kératiniques, comprenant au moins un précurseur de colorants, au moins un agent alcalin et au moins un composé oxydant, **caractérisée en ce qu'**elle comprend en outre :
- un hydrolysat de protéine,
- un copolymère quaternisé de di-méthylallyl ammonium et d'acide acrylique,
- un di-alkyl ester de N méthyl triéthanolamine méthylsulfate, et
- un di-alkyl carbonate.

2. Composition selon la revendication 1, **caractérisée en ce que** l'hydrolysat de protéine, le copolymère quaternisé de diméthylallyl ammonium et d'acide acrylique, le di-alkyl ester de N méthyl triéthanolamine méthylsulfate et le di-alkyl carbonate forment un complexe associatif quaternaire au sein de la composition.

3. Composition selon l'une des revendications 1 et 2, **caractérisée en ce que** l'hydrolysat de protéine est un hydrolysat de séricine.

4. Composition selon l'une quelconque des revendications précédentes **caractérisé en ce que** le copolymère quaternisé de diméthylallyl ammonium et d'acide acrylique répond à la formule I suivante : 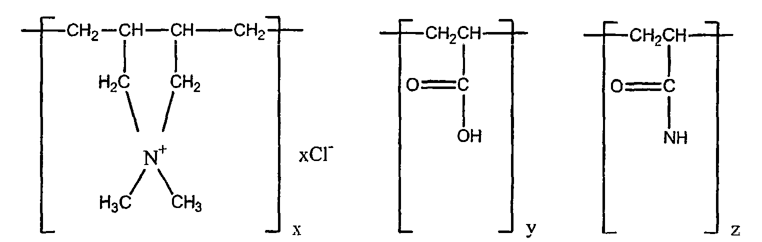 où x est compris entre 1 et 1000, de préférence entre 1 et 100, et y et z sont compris indépendamment entre 0 et 1000, de préférence entre 0 et 100.

5. Composition selon l'une quelconque des revendications 1 à 4, **caractérisée en ce que** le copolymère quaternisé de di-méthylallyl ammonium et d'acide acrylique est un polyquaternium, de préférence un polyquaternium 22.

6. Composition selon l'une quelconque des revendications précédentes **caractérisé en ce que** le di-alkyl ester de N méthyl triéthanolamine méthylsulfate répond à la formule II suivante : 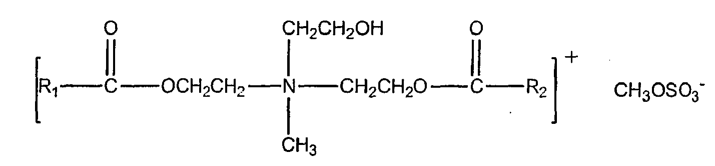 où R₁ et R₂ sont des groupes alkyles ou alcényles en Cₙ, où n est compris entre 1 et 100, de préférence entre 6 et 20.

7. Composition selon la revendication 6 **caractérisé en ce que** les groupes R₁ et R₂ sont choisis parmi les groupes caproyle, caprylyle, capryle, lauryle, myristyle, cétyle, stéaryle, arachidyle, béhényle, caproléyle, lauroléyle, myristoléyle, palmitoléyle, oléyle, linoléyle, linolényle, arachidonyle et érucyle.

8. Composition selon l'une quelconque des revendications 1 à 7, **caractérisée en ce que** le di-alkyl ester de N méthyl triéthanolamine méthylsulfate est un dicocoyl ester de N méthyl tri éthanolamine méthylsulfate.

9. Composition selon l'une quelconque des revendications précédentes **caractérisée en ce que** di-alkyl carbonate répond à la formule III suivante : 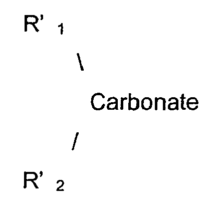 où R'₁ et R'₂ sont des groupes alkyles ou alcényles en Cₙ, linéaires ou ramifiés, où n est compris entre 1 et 100, de préférence entre 6 et 20.

10. Composition selon la revendication 9 **caractérisée en ce que** les groupes R'₁ et R'₂ sont choisis parmi les groupes caproyle, caprilyle, capryle, lauryle, myristyle, cétyle, stéaryle, arachidyle, béhényle, caproléyle, lauroléyle, myristoléyle, palmitoléyle, oléyle, linoléyle, linolényle, arachidonyle et érucyle.

11. Composition selon la revendication 10, **caractérisée en ce que** le di-alkyl carbonate est le di-caprilyl carbonate.

12. Composition selon l'une quelconque des revendications 1 à 11, **caractérisée en ce que** le composé oxydant est le peroxyde d'hydrogène.

13. Composition selon l'une des revendications 1 à 12, **caractérisée en ce que** le ou les agents alcalins sont choisis parmi l'aminométhylpropanol, la monoéthanolamine, la diéthanolamine, la triéthanolamine, l'ammoniaque et leurs mélanges.

14. Composition selon l'une quelconque des revendications précédentes **caractérisée en ce que** le ou les précurseurs de colorants sont choisis parmi les diamines aromatiques, les aminophénols et les phénols.

15. Composition selon la revendication 14 **caractérisé en ce que** le ou les précurseurs de colorants sont choisis parmi la para-phénylène diamine (pPD), l'ortho-aminophénol (oAP), le para-méthylamino phénol (pMAP), le para-amino phénol (pAP), la para-toluylène diamine (pTD), la N phényl paraphénylène diamine (NpPD), le méta-aminophénol (mPD), le résorcinol (R), le 1-naphtol (1-N), la méta-phénylène diamine (mPD), le para-amino ortho-crésol (pAOC), le 1-5 dihydroxynaphtalène (1-5 DHN) et/ou le 2-7 dihydroxynaphtalène (2-7 DHN).

16. Composition selon l'une quelconque des revendications précédentes, **caractérisée en ce que** le pH est compris entre 7 et 11, de préférence entre 8,5 et 10,5.

17. Composition selon l'une quelconque des revendications précédentes **caractérisée en ce que** l'hydrolysat de protéine représente de 0,1 à 10%, de préférence de 1 à 5%, en poids du poids total de la composition.

18. Composition selon l'une quelconque des revendications précédentes **caractérisée en ce que** le copolymère quaternisé de diméthylallyl ammonium et d'acide acrylique représente de 0,1 à 1,5%, de préférence de 0,5 à 1 %, en poids du poids total de la composition.

19. Composition selon l'une quelconque des revendications précédentes **caractérisée en ce que** le di-alkyl ester de N méthyl triéthanolamine méthylsulfate représente de 0,1 à 5%, de préférence de 0,5 à 2%, en poids du poids total de la composition.

20. Composition selon l'une quelconque des revendications précédentes **caractérisée en ce que** le di-alkyl carbonate représente de 0,02 à 2,5%, de préférence de 0,1 à 1 %, en poids du poids total de la composition.

21. Composition selon l'une quelconque des revendications précédentes **caractérisée en ce que** le ou les composés oxydants représentent de 0,75 à 6%, de préférence de 3 à 4,5%, en poids du poids total de la composition.

22. Composition selon l'une quelconque des revendications précédentes **caractérisée en ce que** le ou les agents alcalins représentent de 1 à 30%, de préférence de 1 à 4%, en poids du poids total de la composition.

23. Composition selon l'une quelconque des revendications précédentes **caractérisée en ce que** le ou les précurseurs de colorants représentent de 0,5 à 10%, de préférence de 1 à 2,5%, en poids du poids total de la composition.

24. Composition selon l'une quelconque des revendications précédentes, **caractérisée en ce qu'**elle comprend :
- entre 0,1 et 10% en poids d'hydrolysat de protéine, de préférence d'hydrolysat de séricine,
- entre 0,1 et 1,5% en poids de copolymère quaternisé de diméthylallyl ammonium et d'acide acrylique,
- entre 0,1 et 5% en poids de di-alkyl ester de N méthyl triéthanolamine méthylsulfate,
- entre 0,02 et 2,5% en poids de di-alkyl carbonate,
- entre 0,75 et 6% en poids d'au moins un composé oxydant,
- entre 0,5 et 10% en poids d'au moins un précurseur de colorants, et
- une quantité suffisante d'au moins un agent alcalin pour atteindre un pH compris entre 7 et 11, de préférence entre 8,5 et 10,5.

25. Composition selon l'une quelconque des revendications précédentes, **caractérisée en ce qu'**elle comprend :
- entre 1 et 5% en poids d'hydrolysat de protéine, de préférence d'hydrolysat de séricine,
- entre 0,5 et 1% en poids de copolymère quaternisé de diméthylallyl ammonium et d'acide acrylique,
- entre 0,5 et 2% en poids de di-alkyl ester de N méthyl triéthanolamine méthylsulfate,
- entre 0,1 et 1 % en poids de di-alkyl carbonate,
- entre 3 et 4,5% en poids d'au moins un composé oxydant,
- entre 1 et 2,5% en poids d'au moins un précurseur de colorants, et
- une quantité suffisante d'au moins un agent alcalin pour atteindre un pH compris entre 7 et 11, de préférence entre 8,5 et 10,5.

26. Composition selon l'une quelconque des revendications précédentes **caractérisée en ce qu'**elle comprend des adjuvants choisis parmi les acides gras, naturels ou synthétiques, les alcools gras, naturels ou synthétiques, oxyéthylénés ou polyglycérolés, les huiles minérales ou végétales, les agents anti-oxydants, les séquestrants et les parfums.

27. Composition selon la revendication 26 **caractérisée en ce que** les acides gras représentent de 1 à 20%, de préférence de 5 à 15%, en poids du poids total de la composition.

28. Composition selon la revendication 26 **caractérisée en ce que** les alcools gras représentent de 1 à 25%, de préférence de 5 à 20%, en poids du poids total de la composition.

29. Composition selon l'une quelconque des revendications précédentes, **caractérisée en ce qu'**elle se présente sous la forme d'une crème ou d'un gel, de préférence sous la forme d'une crème.

30. Complexe associatif quaternaire pour la coloration de fibres kératiniques, **caractérisé en ce qu'**il comprend :
- un hydrolysat de protéine, de préférence un hydrolysat de séricine,
- un copolymère quaternisé de di-méthylallyl ammonium et d'acide acrylique,
- un di-alkyl ester de N méthyl triéthanolamine méthylsulfate et,
- un di-alkyl carbonate.

31. Base, se présentant sous la forme d'un gel ou d'une crème, pour la fabrication d'une composition selon l'une quelconque des revendications 1 à 29, **caractérisée en ce qu'**elle comprend :
- un hydrolysat de protéine, de préférence un hydrolysat de séricine,
- un copolymère quaternisé de di-méthylallyl ammonium et d'acide acrylique,
- un di-alkyl ester de N méthyl triéthanolamine méthylsulfate,
- un di-alkyl carbonate,
- au moins un précurseur de colorants et,
- au moins un agent alcalin.

32. Procédé pour révéler la coloration d'une composition selon l'une quelconque des revendications 1 à 29, **caractérisé en ce qu'**il comprend l'étape de mélange de la base définie à la revendication 31 avec une solution comprenant au moins un composé oxydant, de préférence le peroxyde d'hydrogène.

33. Procédé selon la revendication 32, **caractérisé en ce que** le ou les composés oxydants représentent de 1,5 à 12 %, de préférence de 6 à 9 %, en poids du poids de ladite solution.

34. Procédé selon la revendication 32 ou 33 **caractérisé en ce que** ladite base et ladite solution sont mélangées dans un rapport 1/1 en poids.

35. Procédé de coloration de fibres kératiniques, de préférence de cheveux, comprenant les étapes suivantes :
- application sur lesdites fibres d'une composition selon l'une quelconque des revendications 1 à 29, pendant une durée permettant d'obtenir la coloration recherchée, de préférence pendant 10 à 45 minutes, mieux de l'ordre de 30 minutes,
- rinçage et séchage des fibres.

## Claims

1. A composition for permanent dyeing of keratinous fibres, comprising at least one dye precursor, at least one alkaline agent, and at least one oxidizing compound, **characterized in that** it further comprises:
a protein hydrolyzate,
a quaternized copolymer of di-methylallyl ammonium and acrylic acid,
a N-methyl triethanolamine methylsulphate di-alkyl ester, and
a di-alkyl carbonate.

2. A composition according to claim 1, **characterized in that** the protein hydrolyzate, the quaternized copolymer of dimethylallyl ammonium and acrylic acid, the N-methyl triethanolamine methylsulphate di-alkyl ester and the di-alkyl carbonate form a quaternary associative complex within the composition.

3. A composition according to one of claims 1 and 2, **characterized in that** the protein hydrolyzate is a sericin hydrolyzate.

4. A composition according to any one of the preceding claims, **characterized in that** the quaternized copolymer of di-methylallyl ammonium and acrylic acid has the following formula I: 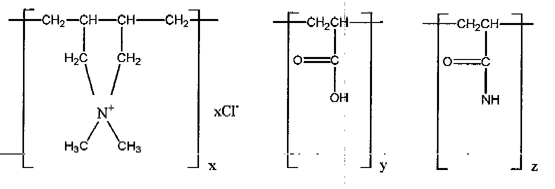 wherein x ranges from 1 to 1,000, preferably from 1 to 100, and y and z independently range from 0 to 1,000, preferably from 0 to 100.

5. A composition according to any one of claims 1 to 4, **characterized in that** the quaternized copolymer of di-methylallyl ammonium and acrylic acid is a polyquaternium, preferably a polyquaternium 22.

6. A composition according to any one of the preceding claims, **characterized in that** the N methyl triethanolamine methylsulphate di-alkyl ester has the following formula II: 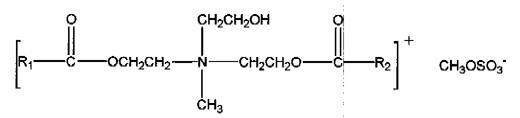 wherein R₁ and R₂ are Cₙ alkyl or alkenyl groups, wherein n ranges from 1 to 100, preferably from 6 to 20.

7. A composition according to claim 6, **characterized in that** R₁ and R₂ are selected amongst caproyl, caprylyl, capryl, lauryl, myristyl, cetyl, stearyl, arachidyl, behenyl, caproleyl, lauroleyl, myristoleyl, palmitolyl, oleyl, linoleyl, linolenyl, arachidonyl and erucyl groups.

8. A composition according to any one of claims 1 to 7, **characterized in that** the N-methyl triethanolamine methylsulphate di-alkyl ester is a N- methyl triethanolamine methylsulphate dicocoyl ester.

9. A composition according to any one of the preceding claims, **characterized in that** the di-alkyl carbonate has the following formula III : 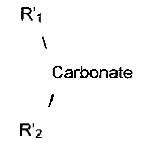 wherein R'₁ and R'₂ are Cₙ, straight or branched, alkyl or alkenyl groups, where n ranges from 1 to 100, preferably from 6 to 20.

10. A composition according to claim 9, **characterized in that** R'₁ and R'₂ are selected amongst caproyl, capryilyl, capryl, lauryl, myristyl, cetyl, stearyl, arachidyl, behenyl, caproleyl, lauroleyl, myristoleyl, palmitoleyl, oleyl, linoleyl, linolenyl, arachidonyl and erucyl groups.

11. A composition according to claim 10, **characterized in that** the di-alkyl carbonate is di-caprilyl carbonate.

12. A composition according to any one of claims 1 to 11, **characterized in that** the oxidizing compound is hydrogen peroxide.

13. A composition according to any one of claims 1 to 12, **characterized in that** the alkaline agent(s) is/are selected from the group consisting of aminomethylpropanol, monoethanolamine, diethanolamine, triethanolamine, ammonia and the mixtures thereof.

14. A composition according to any one of the preceding claims, **characterized in that** the dye precursor(s) is/are selected amongst aromatic diamines, aminophenols and phenols.

15. A composition according to claim 14, **characterized in that** the dye precursor(s) is/are selected amongst para-phenylene diamine (pPD), ortho-aminophenol (oAP), para-methylamino phenol (pMAP), para-amino phenol (pAP), para-toluene diamine (pTD), N-phenyl paraphenylene diamine (NpPD), meta-aminophenol (mPD), resorcinol (R), 1-naphthol(1-N), meta-phenylene diamine (mPD), para-amino ortho-cresol (pAOC), 1,5- dihydroxynaphthalene (1,5-DHN) and/or 2,7 dihydroxynaphtalene (2,7- DHN).

16. A composition according to any one of preceding claims, **characterized in that** its pH ranges from 7 to 11, preferably from 8.5 to 10.5.

17. A composition according to any one of preceding claims, **characterized in that** the protein hydrolyzate represents from 0.1 to 10%, more preferably from 1 to 5% in weight based on the total weight of the composition.

18. A composition according to any one of preceding claims, **characterized in that** the quaternized copolymer of di-methylallyl ammonium and acrylic acid represents from 0.1 to 1.5%, more preferably from 0.5 to 1%, in weight based on the total weight of the composition.

19. A composition according to any one of preceding claims, **characterized in that** N-methyl triethanolamine methylsulphate di-alkyl ester represents from 0.1 to 5%, more preferably from 0.5 to 2%, in weight based on the total weight of the composition.

20. A composition according to any one of the preceding claims, **characterized in that** the di-alkyl carbonate represents from 0.02 to 2.5%, preferably from 0.1 to 1 % in weight based on the total weight of the composition.

21. A composition according to any one of the preceding claims, **characterized in that** the oxidizing compound(s) represent(s) from 0.75 to 6%, preferably from 3 to 4.5% in weight, based on the total weight of the composition

22. A composition according to any one of the preceding claims, **characterized in that** the alkaline agent(s) represent(s) from 1 to 30%, preferably from 1 to 4% in weight based on the total weight of the composition.

23. A composition according to any one of the preceding claims, **characterized in that** the dye precursor(s) represent(s) from 0.5 to 10%, preferably from 1 to 2.5% in weight based on the total weight of the composition.

24. A composition according to any one of the preceding claims, **characterized in that** it comprises:
- from 0.1 to 10% in weight of a protein hydrolyzate, preferaby a sericin hydrolyzate,
- from 0.1 to 1.5% in weight of a quaternized copolymer of di-methylallyl ammonium and acrylic acid,
- from 0.1 to 5% in weight of N-methyl triethanolamine methylsulphate di-alkyl ester,
- from 0.02 to 2,5% in weight of di-alkyl carbonate,
- from 0.75 to 6% in weight of at least one oxidizing compound,
- from 0.5 to 10% in weight of at least one dye precursor, and
- a sufficient amount of at least one alkaline agent for reaching a pH ranging from 7 to 11, preferably from 8.5 to 10.5.

25. A composition according to any one of the preceding claims, **characterized in that** it comprises:
- from 1 to 5% in weight of a protein hydrolyzate, preferably a sericin hydrolyzate,
- from 0.5 to 1% in weight of a quaternized copolymer of di-methylallyl ammonium and acrylic acid,
- from 0.5 to 2% in weight of N-methyl triethanolamine methylsulphate di-alkyl ester,
- from 0,1 to 1% in weight of di-alkyl carbonate,
- from 3 to 4.5% in weight of at least one oxidizing compound,
- from 1 to 2.5% in weight of at least one dye precursor, and
- a sufficient amount of at least one alkaline agent for reaching a pH ranging from 7 to 11, preferably from 8.5 to 10.5.

26. A composition according to any one of the preceding claims, **characterized in that** it comprises adjuvants selected amongst naturally occurring or synthetic fatty acids, naturally occurring or synthetic fatty alcohols, either oxyethylenated or polyglycerolated, mineral or vegetable oils, antioxidizing agents, sequestering agents and fragrances.

27. A composition according to claim 26, **characterized in that** the fatty acids represent from 1 to 20%, preferably from 5 to 15% in weight based on the total weight of the composition.

28. A composition according to claim 26, **characterized in that** the fatty alcohols represent from 1 to 25%, preferably from 5 to 20% in weight based on the total weight of the composition.

29. A composition according to any one of the preceding claims, **characterized in that** it is in the form of a cream or a gel, preferably in the form of a cream.

30. A quaternary associative complex for dyeing keratinous fibres, **characterized in that** it comprises:
- a protein hydrolyzate, preferably a sericin hydrolyzate,
- a quaternized copolymer of di-methylallyl ammonium and acrylic acid,
- a N-methyl triethanolamine methylsulphate di-alkyl ester, and
- a di-alkyl carbonate.

31. A base, having the form of a gel or a cream, for preparing a composition according to any one of claims 1 to 29, **characterized in that** it comprises:
- a protein hydrolyzate, preferably a sericin hydrolyzate,
- a quaternized copolymer of di-methylallyl ammonium and acrylic acid,
- a N-methyl triethanolamine methylsulphate di-alkyl ester,
- a di-alkyl carbonate.
- at least one dye precursor, and
- at least one alkaline agent.

32. A method for revealing the composition dye according to any one of claims 1 to 29, **characterized in that** it comprises a step involving mixing the base such as defined in claim 31 with a solution comprising at least one oxidizing compound, preferably hydrogen peroxide.

33. A method according to claim 32, **characterized in that** the oxidizing compound(s) represent(s) from 1.5 to 12%, preferably from 6 to 9%, in weight based on the weight of said solution.

34. A method according to claim 32 or 33 **characterized in that** said base and said solution are mixed in a 1/1 weight ratio.

35. A method for dyeing keratinous fibres, preferably hair, comprising the following steps of:
- applying onto said fibres a composition according to any one of claims 1 to 29, for a period of time sufficient for obtaining the desired colouring, preferably from 10 to 45 minutes, more preferably in the order of 30 minutes,
- rinsing and drying of the fibres.

## Patentansprüche

1. Zusammensetzung zur dauerhaften Färbung von Keratinfasern, umfassend mindestens eine Färbemittel-Vorstufe, mindestens ein alkalisches Mittel und mindestens eine oxidierende Verbindung, **dadurch gekennzeichnet, dass** sie außerdem Folgendes umfasst.
- ein Proteinhydrolysat,
- ein quaternisiertes Copolymer von Dimethylallylammonium und Acrylsäure,
- einen Dialkylester von N-Methyltriethanolaminmethylsulfat und
- ein Dialkylcarbonat.

2. Zusammensetzung nach Anspruch 1, **dadurch gekennzeichnet, dass** das Proteinhydrolysat, das quaternisierte Copolymer von Dimethylallytammonium und Acrylsäure, der Dialkylester von N-Methyltriethanolaminmethylsulfat und das Dialkylcarbonat einen assoziativen quaternären Komplex in der Zusammensetzung bilden.

3. Zusammensetzung nach einem der Ansprüche 1 und 2, **dadurch gekennzeichnet, dass** das Proteinhydrolysat ein Sericinhydrolysat ist.

4. Zusammensetzung nach einem der vorhergehenden Ansprüche, **dadurch gekennzeichnet, dass** das quaternisierte Copolymer von Dimethylallylammonium und Acrylsäure der folgenden Formel I entspricht: 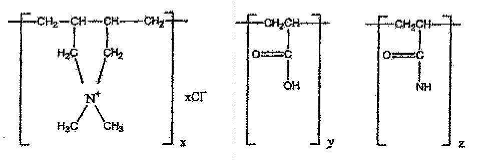 wobei x zwischen 1 und 1000, vorzugsweise zwischen 1 und 100 liegt und y und z unabhängig zwischen 0 und 1000, vorzugsweise zwischen 0 und 100 liegen.

5. Zusammensetzung nach einem der Ansprüche 1 bis 4, **dadurch gekennzeichnet, dass** das quaternisierte Copolymer von Dimethylallylammonium und Acrylsäure ein Polyquaternium, vorzugsweise ein Polyquaternium 22 ist.

6. Zusammensetzung nach einem der vorhergehenden Ansprüche, **dadurch gekennzeichnet, dass** der Dialkylester von N-Methyltriethanolaminmethylsulfat der folgenden Formel II entspricht: 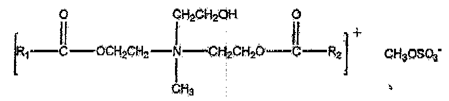 wobei R₁ und R₂ Alkyl- oder Alkenylgruppen mit Cₙ sind, wobei n zwischen 1 und 100, vorzugsweise zwischen 6 und 20 liegt.

7. Zusammensetzung nach Anspruch 6, **dadurch gekennzeichnet, dass** die Gruppen R₁ und R₂ aus der Caproyl-, Caprylyl-, Caryl-, Lauryl-, Myristyl-, Cetyl-, Stearyl-, Arachidyl-, Behenyl-, Caproleyl-, Lauroleyl-, Myristoleyl-, Palmitoleyl-, Oleyl-, Linoleyl-, Linolenyl-, Arachindonyl- und Erucylgruppe ausgewählt sind.

8. Zusammensetzung nach einem der Ansprüche 1 bis 7, **dadurch gekennzeichnet, dass** der Dialkylester von N-Methyltriethanolaminmethylsulfat ein Dicocoylester von N-Methyltriethanolaminmethylsulfat ist.

9. Zusammensetzung nach einem der vorhergehenden Ansprüche, **dadurch gekennzeichnet, dass** das Dialkylcarbonat der folgenden Formel III entspricht: 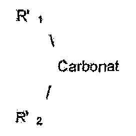 wobei R'₁ und R'₂ lineare oder verzweigte Alkyl- oder Alkenylgruppen mit Cₙ sind, wobei n zwischen 1 und 100, vorzugsweise zwischen 6 und 20 liegt.

10. Zusammensetzung nach Anspruch 9, **dadurch gekennzeichnet, dass** die Gruppen R'₁ und R'₂ aus der Caproyl-, Caprylyl-, Caryl-, Lauryl-, Myristyl-, Cetyl-, Stearyl-, Arachidyl-, Behenyl-, Caproleyl-, Lauroleyl-, Myristoleyl-, Palmitoleyl-, Oleyl-, Linoleyl-, Liriolenyl-, Arachindonyl- und Erucylgruppe ausgewählt sind.

11. Zusammensetzung nach Anspruch 10, **dadurch gekennzeichnet, dass** das Dialkylcarbonat Dicaprilylcarbonat ist.

12. Zusammensetzung nach einem der Ansprüche 1 bis 11, **dadurch gekennzeichnet, dass** die oxidierende Verbindung Wasserstoffperoxid ist.

13. Zusammensetzung nach einem der Ansprüche 1 bis 12, **dadurch gekennzeichnet, dass** das alkalische Mittel oder die alkalischen Mittel aus Aminomethylpropanol, Monoethanolamin, Diethanolamin, Triethanolamin, Ammoniak und Mischungen davon ausgewählt ist bzw. sind.

14. Zusammensetzung nach einem der vorhergehenden Ansprüche, **dadurch gekennzeichnet, dass** die Färbemittel-Vorstufe oder die Färbemittel-Vorstufen aus aromatischen Diaminen, Aminophenolen und Phenolen ausgewählt ist bzw. sind.

15. Zusammensetzung nach Anspruch 14, **dadurch gekennzeichnet, dass** die Färbemittel-Vorstufe(n) aus para-Phenylendiamin (pPD), ortho-Aminophenol (oAP), para-Methylaminophenol (pMAP, para-Aminophenol (pAP), para-Toluylendiamin (pTD) N-Phenylparaphenylendiamin (NpPD), meta-Aminophenol (mPD), Resorcin (R), 1-Naphthol (1-N), meta-Phenylendiamin (mPD), para-Amino-ortho-kresol (pAOC), 1,5-Dihydroxynaphthalin (1-5 DHN) und/oder 2,7-Dihydroxynaphthalin (2-7 DHN) ausgewählt Ist (sind).

16. Zusammensetzung nach einem der vorhergehenden Ansprüche, **dadurch gekennzeichnet, dass** ihr pH-Wert zwischen 7 und 11, vorzugsweise zwischen 8,5 und 10,5 liegt.

17. Zusammensetzung nach einem der vorhergehenden Ansprüche, **dadurch gekennzeichnet, dass** das Proteinhydrolysat von 0,1 bis 10 Gew.-%, vorzugsweise von 1 bis 5 Gew.-%, bezogen auf das Gesamtgewicht der Zusammensetzung, darstellt.

18. Zusammensetzung nach einem der vorhergehenden Ansprüche, **dadurch gekennzeichnet, dass** das quaternisierte Copolymer von DImethylallylammonium und Acrylsäure von 0,1 bis 1,5 Gew.-%, vorzugsweise von 0,5 bis 1 Gew.-%, bezogen auf das Gesamtgewicht der Zusammensetzung, darstellt.

19. Zusammensetzung nach einem der vorhergehenden Ansprüche, **dadurch gekennzeichnet, dass** der Dialkylester von N-Methyltriethanolaminmethylsulfat von 0,1 bis 5 Gew.-%, vorzugsweise von 0,5 bis 2 Gew.-%, bezogen auf das Gesamtgewicht der Zusammensetzung, darstellt.

20. Zusammensetzung nach einem der vorhergehenden Ansprüche, **dadurch gekennzeichnet, dass** das Dialkylcarbonat von 0,02 bis 2,5 Gew.-%, vorzugsweise von 0,1 bis 1 Gew.-%, bezogen auf das Gesamtgewicht der Zusammensetzung, darstellt.

21. Zusammensetzung nach einem der vorhergehenden Ansprüche, **dadurch gekennzeichnet, dass** die oxidierende Verbindung oder die oxidierenden Verbindungen 0,75 bis 6 Gew.-%, vorzugsweise 3 bis 4,5 Gew.-%, bezogen auf das Gesamtgewicht der Zusammensetzung, darstellt bzw. darstellen.

22. Zusammensetzung nach einem der vorhergehenden Ansprüche, **dadurch gekennzeichnet, dass** das alkalische Mittel oder die alkalischen Mittel von 1 bis 30 Gew.-%, vorzugsweise von 1 bis 4 Gew.-%, bezogen auf das Gesamtgewicht der Zusammensetzung, darstellt bzw. darstellen.

23. Zusammensetzung nach einem der vorhergehenden Ansprüche, **dadurch gekennzeichnet, dass** die Färbemittelvorstufe oder die Färbemittelvorstufen von 0,5 bis 10 Gew.-%, vorzugsweise von 1 bis 2,5 Gew.-%, bezogen auf das Gesamtgewicht der Zusammensetzung, darstellt bzw. darstellen.

24. Zusammensetzung nach einem der vorhergehenden Ansprüche, **dadurch gekennzeichnet, dass** sie Folgendes umfasst:
- zwischen 0,1 und 10 Gew.-% Proteinhydrolysat, vorzugsweise Sericinhydrolysat,
- zwischen 0,1 und 1,5 Gew.-% quaternisiertes Copolymer von Dimethylallylammonium und Acrylsäure,
- zwischen 0,1 und 5 Gew.-% Dialkylester von N-Methyltriethanolaminmethylsulfat,
- zwischen 0,02 und 2,5 Gew.-% Dialkylcarbonat,
- zwischen 0,75 und 6 Gew,-% mindestens einer oxydierenden Verbindung,
- zwischen 0,5 und 10 Gew.-% mindestens einer Färbemittel-Vorstufe und
- eine Menge mindestens eines alkalischen Mittels, die ausreichend ist, um einen pH-Wert zwischen 7 und 11, vorzugsweise zwischen 8,5 und 10,5 zu erreichen.

25. Zusammensetzung nach einem der vorhergehenden Ansprüche, **dadurch gekennzeichnet, dass** sie Folgendes umfasst:
- zwischen 1 und 5 Gew.-% Proteinhydrolysat, vorzugsweise Sericinhydrolysat,
- zwischen 0,5 und 1 Gew.-% quaternisiertes Copolymer von Dimethylallylammonium und Acrylsäure,
- zwischen 0,5 und 2 Gew.-% Dialkylester von N-Methyltriethanolaminmethylsulfat,
- zwischen 0,1 und 1 Gew.-% Dialkylcarbonat,
- zwischen 3 und 4,5 Gew.-% mindestens einer oxydierenden Verbindung,
- zwischen 1 und 2,5 Gew.-% mindestens einer Färbemittel-Vorstufe und
- eine Menge mindestens eines alkalischen Mittels, die ausreichend ist, um einen pH-Wert zwischen 7 und 11, vorzugsweise zwischen 8,5 und 10,5 zu erreichen.

26. Zusammensetzung nach einem der vorhergehenden Ansprüche, **dadurch gekennzeichnet, dass** sie Hilfsmittel umfasst, die aus natürlichen oder synthetischen Fettsäuren, natürlichen oder synthetischen Fettalkoholen, Oxyethylenen oder Polyglycerinen, mineralischen oder pflanzlichen Ölen, Antioxidantien, Komplexbildnern und Duftstoffen ausgewählt sind.

27. Zusammensetzung nach Anspruch 26, **dadurch gekennzeichnet, dass** die Fettsäuren von 1 bis 20 Gew.-%, vorzugsweise von 5 bis 15 Gew.-%, bezogen auf das Gesamtgewicht der Zusammensetzung, darstellen.

28. Zusammensetzung nach Anspruch 26, **dadurch gekennzeichnet, dass** die Fettalkohole von 1 bis 25 Gew.-%, vorzugsweise von 5 bis. 20 Gew.-%, bezogen auf das Gesamtgewicht der Zusammensetzung, darstellen.

29. Zusammensetzung nach einem der vorhergehenden Ansprüche, **dadurch gekennzeichnet, dass** sie in Form einer Creme oder eines Gels, vorzugsweise In Form einer Creme vorliegt.

30. Assoziativer quaternärer Komplex zur Färbung von Keratinfasern, **dadurch gekennzeichnet, dass** er Folgendes umfasst:
- ein Proteinhydrolysat, vorzugsweise ein Sericinhydrolysat,
- ein quaternisiertes Copolymer von Dimethylallylammonium und Acrylsäure,
- einen Dialkylester von N-Methyltriethanolaminmethylsulfat und
- ein Dialkylcarbonat.

31. Grundstoff, der In Form eines Gels oder einer Creme vorliegt, zur Herstellung einer Zusammensetzung nach einem der Ansprüche 1 bis 29, **dadurch gekennzeichnet, dass** er Folgendes umfasst:
- ein Proteinhydrolysat, vorzugsweise ein Sericinhydrolysat,
- ein quaternisiertes Copolymer von Dimethylallylammonium und Acrylsäure,
- einen Dialkylester von N-Methyltriethanolaminmethylsulfat,
- ein Dialkylcarbonat,
- mindestens eine Färbemittel-Vorstufe und
- mindestens ein alkalisches Mittel.

32. Verfahren zur Entwicklung der Färbung einer Zusammensetzung nach einem der Ansprüche 1 bis 29, **dadurch gekennzeichnet, dass** es den Schritt des Mischens des in Anspruch 31 definierten Grundstoffs mit einer Lösung, die mindestens eine oxidierende Verbindung, vorzugsweise Wasserstoffperoxid, umfasst, umfasst.

33. Verfahren nach Anspruch 32, **dadurch gekennzeichnet, dass** die oxidierende Verbindung oder die oxidierenden Verbindungen 1,5 bis 12 Gew.-%, vorzugsweise 6 bis 9 Gew.-%, bezogen auf das Gewicht der Lösung, darstellen.

34. Verfahren nach Anspruch 32 oder 33, **dadurch gekennzeichnet, dass** der Grundstoff und die Lösungen in einem Verhältnis von 1/1, bezogen auf das Gewicht, vermischt sind.

35. Verfahren zur Färbung von Keratinfasern, vorzugsweise von Haaren, umfassend die folgenden Schritte:
- Auftragen einer Zusammensetzung nach einem der Ansprüche 1 bis 29 auf die Fasern für eine Dauer, die den Erhalt einer gewünschten Färbung ermöglicht, vorzugsweise für 10 bis 45 min, noch mehr bevorzugt in der Größenordnung von 30 min,
- Spülen und Trocknen der Fasern.
